# EUROPEAN PATENT APPLICATION

(11) **EP 4 567 414 A1**
(43) Date of publication of application: **11.06.2025**
(21) Application number: 23849194.8
(22) Date of filing: 14.07.2023
(51) Int. Cl.: G01N 27/02, G01D 11/00

(54) **MEASUREMENT ASSEMBLY, SENSING APPARATUS, AND MANUFACTURING METHOD FOR SENSING APPARATUS**

(30) Priority: 02.08.2022 CN 202210918950; 30.09.2022 CN 202211211162; 31.10.2022 CN 202211342263
(71) Applicant: Hangzhou Sanhua Research Institute Co., Ltd., Hangzhou, Zhejiang 310018 (CN)
(72) Inventor: CHENG, Dezhi, Hangzhou, Zhejiang 310018 (CN); ZHANG, Yuxiang, Hangzhou, Zhejiang 310018 (CN); YANG, Shanhong, Hangzhou, Zhejiang 310018 (CN); HUANG, Longzhong, Hangzhou, Zhejiang 310018 (CN); JIN, Qihong, Hangzhou, Zhejiang 310018 (CN)
(74) Representative: Ipside
(86) International application number: PCT/CN2023/107432
(87) International publication number: WO 2024/027486

(57) **Abstract**

This application provides a detection assembly, including a lead electrode and a conductive terminal. The conductive terminal has a first terminal end and a second terminal end, with the first terminal end having a first end and the second terminal end having a second end. Along the extension direction of the conductive terminal, the lead electrode and the first terminal end are on the same side of the second tip, and the lead electrode and the second terminal end are on the same side of the first tip; or, the lead electrode and the second terminal end are on different sides of the first tip, and the lead electrode is perpendicular to the extension direction of the conductive terminal. This can reduce deformation of the lead electrode caused by external forces.

## Description

### Technical Field

This application relates to the field of measurement technology, specifically to a detection assembly, a sensor, and a manufacturing method for a sensor.

### Background

A sensor includes a detection assembly, a housing, and a circuit board. The detection assembly includes a detection element, a lead electrode, and a conductive terminal. The detection element is connected to the conductive terminal through the lead electrode and is connected to the detection circuit through the conductive terminal. The housing has a cavity, and the detection element, lead electrode, and part of the conductive terminal are contained within the cavity. The conventional detection assembly is typically prone to deformation under external forces, affecting detection accuracy.

Therefore, there is a need to improve the structure of the sensor.

### Summary

This application provides a detection assembly, a sensor, and a manufacturing method for a sensor that, reducing deformation of the detection assembly.

The first aspect of this application provides a detection assembly, which includes a lead electrode and a conductive terminal. The conductive terminal has a first terminal end and a second terminal end. The first terminal end has a first tip, and the second terminal end has a second tip. Along the extension direction of the conductive terminal, the lead electrode and the first terminal end are on the same side of the second tip. The lead electrode includes a connection portion and an extension portion. The connection portion is connected to the first terminal end, and the extension portion extends from the connection portion in a direction away from the conductive terminal.

Along the extension direction of the conductive terminal, the connection portion and the second terminal end are on the same side of the first tip. Or, the connection portion and the second terminal end are on different sides of the first tip, and the connection portion is perpendicular to the extension direction of the conductive terminal.

Along the extension direction of the conductive terminal, the extension portion and the second terminal end are on the same side of the first tip; or, the extension portion and the second terminal end are on different sides of the first tip, and the extension portion is perpendicular to the extension direction of the conductive terminal.

In the detection assembly of this application, the lead electrode has a connection portion and an extension portion, and the lead electrode and the first terminal end are on the same side of the second tip. Along the extension direction of the conductive terminal, the connection portion and the second terminal end are on the same side of the first tip. the connection portion and the second terminal end are on different sides of the first tip, and the connection portion is perpendicular to the extension direction of the conductive terminal. Along the extension direction of the conductive terminal, the extension portion and the second terminal end are on the same side of the first tip; or, the extension portion and the second terminal end are on different sides of the first tip, and the extension portion is perpendicular to the extension direction of the conductive terminal. As such, the portion of the lead electrode protruding from the conductive terminal is reduced, which can reduce deformation of the lead electrode that may occur under external forces.

The second aspect of this application provides a sensor, including a detection assembly, a housing, and a circuit board. The housing is connected to the circuit board, and the detection assembly is electrically connected to the circuit board. The detection assembly includes a detection element, a lead electrode, and a conductive terminal. The lead electrode is connected to the detection element, and the lead electrode is connected to the conductive terminal.

The conductive terminal includes a first terminal end and a second terminal end. The first terminal end has a first tip, and the second terminal end has a second tip. Along the extension direction of the conductive terminal, the lead electrode and the first terminal end are on the same side of the second tip. The lead electrode includes a connection portion and an extension portion. The connection portion is connected to the first terminal end, and the extension portion extends from the connection portion in a direction away from the conductive terminal.

Along the extension direction of the conductive terminal, the connection portion and the second terminal end are on the same side of the first tip, the connection portion and the second terminal end are on different sides of the first tip, and the connection portion is perpendicular to the extension direction of the conductive terminal;

Along the extension direction of the conductive terminal, the extension portion and the second terminal end are on the same side of the first tip, the extension portion and the second terminal end are on different sides of the first tip, and the extension portion is perpendicular to the extension direction of the conductive terminal;

The housing has a cavity, with the detection element, lead electrode, and first terminal end all located within the cavity, while the second terminal end is located outside the cavity.

In the sensor, along the extension direction of the conductive terminal, both the first side and second side of the detection element are on the same side of the first tip as the second terminal end; or, the first side and the second terminal end are on different sides of the first tip, the second side and the second terminal end are on different sides of the first tip, and the second side is flush with the first tip. the first side and the second side are on different sides of the first tip. As such, the portion of the lead electrode protruding from the conductive terminal is reduced. Accordingly, during assembly of the detection assembly with the housing, potential deformation of the lead electrode due to collision between the detection element and the inner wall of the housing.

The third aspect of this application provides a manufacturing method for a sensor. The method may include providing a detection assembly, a housing, and a circuit board, where the detection assembly includes a detection element, a conductive part, and a base, the detection element is connected to the conductive part, and the conductive part is connected to the base; connecting the detection assembly to the circuit board; and fitting the housing over at least part of the detection assembly, and connecting the housing to the circuit board.

In the manufacturing method for the sensor, the detection assembly and housing are separately connected to the circuit board. Compared to the conventional manufacturing method that first connects the base and housing to form a probe, and then connects the probe and circuit board, the disclosed method simplifies the assembly between the base and the housing, thus simplifying the manufacturing of the sensor.

### Brief Description of The Drawings

Figure 1 is a schematic diagram of a detection assembly in the prior art;
Figure 2 is a schematic diagram of the assembly of a detection assembly and housing in the prior art;
Figure 3 is a front view of an exemplary detection assembly consistent with a first embodiment of this application;
Figure 4 is a rear view of the exemplary detection assembly provided in the first embodiment of this application;
Figure 5 is a perspective view of the exemplary detection assembly provided in the first embodiment of this application;
Figure 6 is an exploded view of the exemplary detection assembly provided in the first embodiment of this application;
Figure 7 is a top view of the exemplary detection assembly provided in the first embodiment of this application;
Figure 8 is a schematic diagram showing an exemplary connection between a lead electrode and a detection element in the first embodiment of this application;
Figure 9 is a front view of an exemplary detection assembly consistent with a second embodiment of this application;
Figure 10 is a rear view of the exemplary detection assembly provided in the second embodiment of this application;
Figure 11 is a perspective view of the exemplary detection assembly provided in the second embodiment of this application;
Figure 12 is a front view of an exemplary detection assembly consistent with a third embodiment of this application;
Figure 13 is a perspective view of the exemplary detection assembly provided in the third embodiment of this application;
Figure 14 is an exploded view of the exemplary detection assembly provided in the third embodiment of this application;
Figure 15 is a top view of the exemplary detection assembly provided in the third embodiment of this application;
Figure 16 is a schematic diagram showing an exemplary connection between the lead electrode and detection element in the third embodiment of this application;
Figure 17 is a perspective view of an exemplary detection assembly consistent with a fourth embodiment of this application;
Figure 18 is a top view of the exemplary detection assembly provided in the fourth embodiment of this application;
Figure 19 is a schematic diagram of an exemplary detection assembly consistent with a fifth embodiment of this application;
Figure 20 is a schematic diagram of an exemplary detection assembly consistent with a sixth embodiment of this application;
Figure 21 is a schematic diagram showing an exemplary detection assembly consistent with the seventh embodiment of this application;
Figure 22 is a schematic diagram of an exemplary connection between a lead electrode and a detection element in the seventh embodiment of this application;
Figure 23 is a schematic diagram of an exemplary assembly of a detection assembly and a housing provided in one embodiment of this application;
Figure 24 is an exploded view of the detection assembly and the housing provided in one embodiment of the application;
Figure 25 is a schematic diagram showing a detection assembly assembled with a housing consistent with one embodiment of this application;
Figure 26 is an exploded view of a detection assembly and a housing consistent with one embodiment of this application;
Figure 27 is a sectional view of an exemplary sensor consistent with one embodiment of this application;
Figure 28 is a schematic diagram showing a first housing and a second housing provided in one embodiment of the application;
Figure 29 is a sectional view of an exemplary sensor consistent with another embodiment of this application;
Figure 30 is a schematic diagram of the exemplary sensor provided in the embodiment of this application;
Figure 31 is a sectional view, from another angle, of the exemplary sensor consistent with the embodiment of this application;
Figure 32 is a sectional view, from yet another angle, of the exemplary sensor provided in the embodiment of this application;
Figure 33 is a schematic diagram showing a first housing and a second housing consistent with the embodiment of this application; and
Figure 34 is a schematic diagram of an exemplary circuit board provided in the embodiment of this application.

### Detailed Description

The embodiments of this application are described in detail in conjunction with the accompanying drawings.

It should be noted that the embodiments described in this application are only exemplary and without limitation. Other embodiments that can be derived by those skilled in the art based on the descriptions of this application without undue experimentation fall within the scope this application.

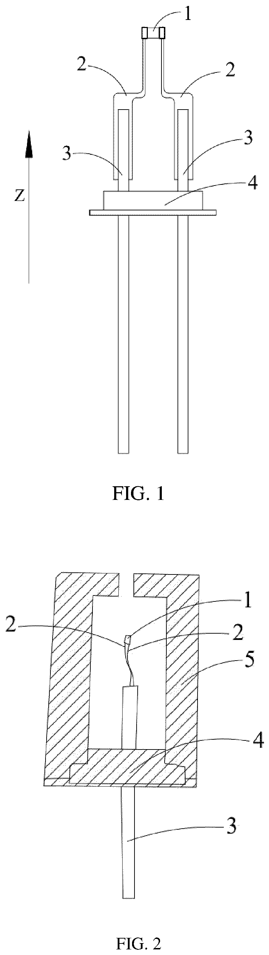

A detection assembly is used to detect gases, environmental temperature, etc. In one example as shown in Figure 1, a detection assembly may include a detection element 1, lead electrodes 2, and conductive terminals 3, where one end of each lead electrode 2 is connected to a corresponding conductive terminal 3, and the other end of each lead electrode 2 is connected to the detection element 1. In a conventional detection assembly, at least part of the lead electrode 2 protrudes from the conductive terminal 3, along the extension direction Z of the conductive terminal 3, as shown in Figure 1 for lead electrode 2. The lead electrode 2 is a flexible component and is prone to deformation under external forces. As another example as shown in Figure 2, a sensor may include a detection assembly and a housing 5. The housing 5 protects the detection assembly to reduce deformation to the detection assembly caused by external forces. As shown in Figure 2, during the assembly of the detection assembly and the housing 5, the detection element 1 and lead electrode 2 protruding from the conductive terminal 3 first enter the inner cavity of the housing 5, and after colliding with the inner wall of the housing 5, the lead electrode 2 becomes twisted and bent. The deformation of the lead electrode 2 not only affects the transmission of the detection signal but also makes it prone to failure in its connection with the detection element 1 or the conductive terminal 3. Additionally, since the lead electrode 2 protrudes from one end of the conductive terminal 3, the overall height of the detection assembly is increased, which is not conducive to the miniaturization of the entire sensor.

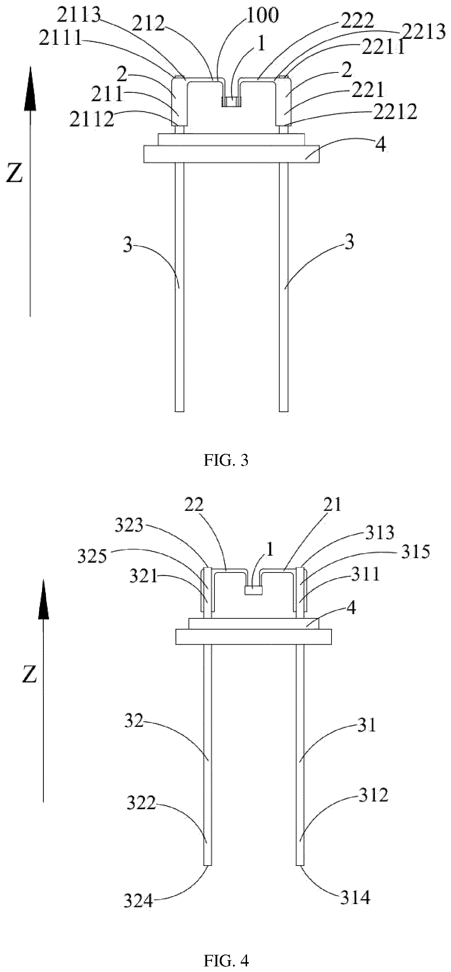

To address this, the first aspect of this application provides a detection assembly, such as a detection assembly 100, to reduce deformation of the lead electrode under external forces. The detection assembly 100 includes a detection element 1, a lead electrode 2, and a conductive terminal 3, where the lead electrode 2 is electrically connected to the conductive terminal 3, and the lead electrode 2 is electrically connected to the detection element 1, as shown in Figure 3. The detection signal from the detection element 1 is transmitted through the lead electrode 2 to the conductive terminal 3, and then through the conductive terminal 3 to the detection circuit.

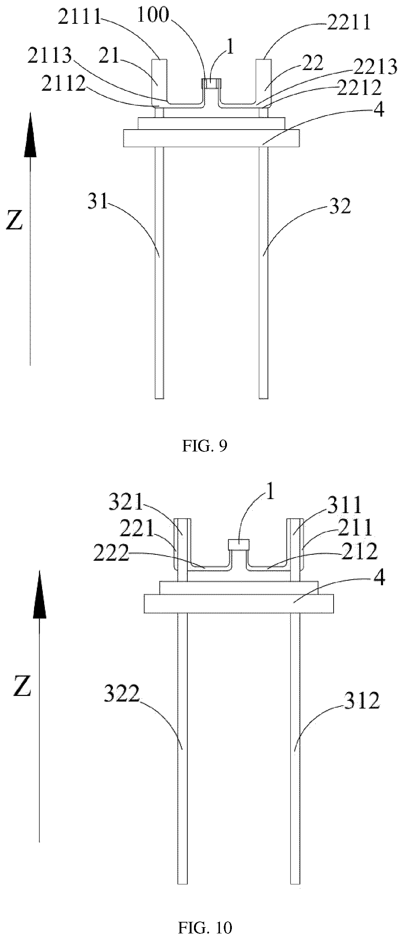

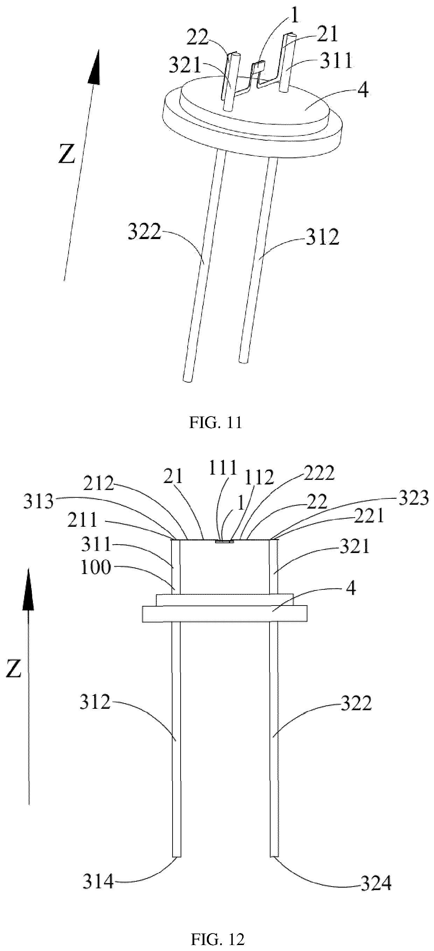

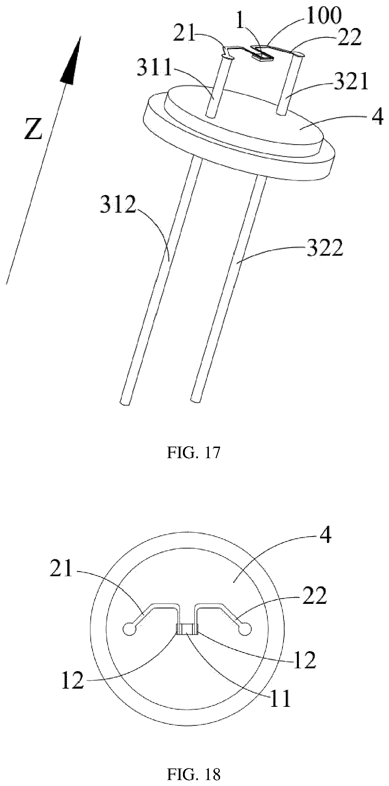

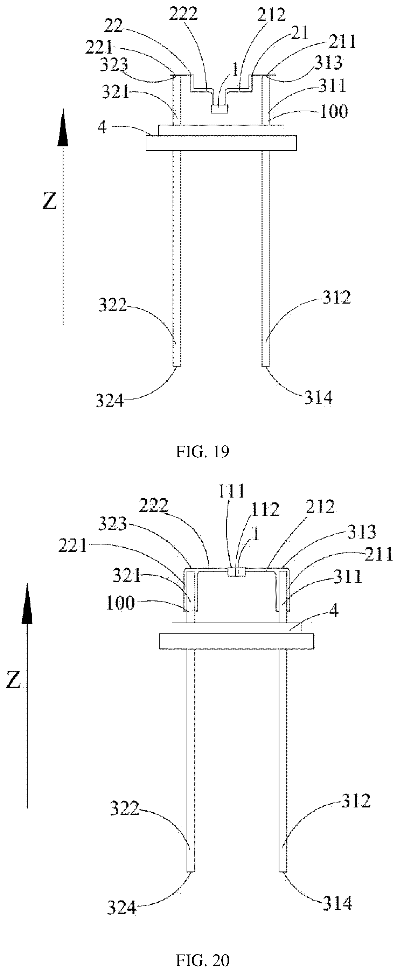

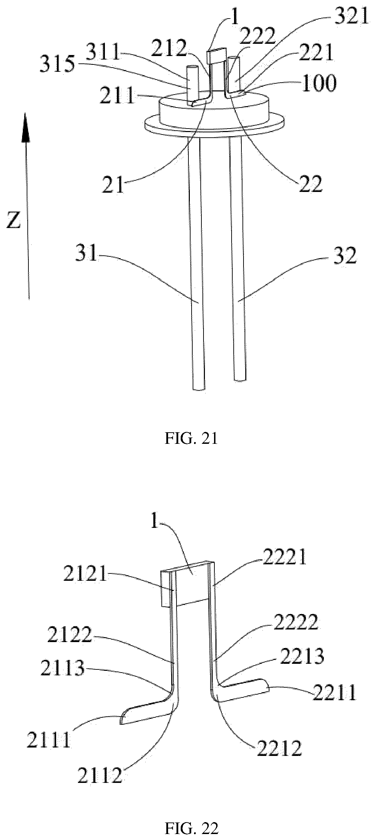

This application provides seven embodiments of the detection assembly 100. For example, Figures 3~8 show schematic diagrams of the first embodiment, Figures 9~11 show schematic diagrams of the second embodiment, Figures 12~16 show schematic diagrams of the third embodiment, Figures 17 and 18 show schematic diagrams of the fourth embodiment, Figure 19 shows a schematic diagram of the fifth embodiment, Figure 20 shows a schematic diagram of the sixth embodiment, and Figures 21 and 22 show schematic diagrams of the seventh embodiment.

The conductive terminal 3 is a rigid component that not only transmits signals but also supports the lead electrode 2, allowing the lead electrode 2 to stand upright, thereby enabling the detection element 1 to fully contact the substance being detected or to be fully exposed to the environment being monitored.

The detection assembly 100 includes at least one pair of lead electrodes 2. The shape, structure, and dimensions of lead electrodes 2 in the pair can be the same or different. For example, as shown in Figures 3 and 4, the detection assembly 100 includes a first lead electrode 21 and a second lead electrode 22 with the same shape, structure, and dimensions, forming a pair of lead electrodes 2.

The detection assembly 100 also includes at least one pair of conductive terminals 3, and the shape, structure, and dimensions of conductive terminals 3 in the pair can be the same or different. For example, as shown in Figures 3~5, the detection assembly 100 includes a first conductive terminal 31 and a second conductive terminal 32 with the same shape, structure, and dimensions, forming a pair of conductive terminals 3. Both the first conductive terminal 31 and the second conductive terminal 32 are columnar, for example, cylindrical.

As shown in Figures 3~5, the first conductive terminal 31 has a first terminal end 311 and a second terminal portion 312. Along the extension direction Z of the conductive terminal 3, the first terminal end 311 and the second terminal portion 312 of the first conductive terminal 31 are located at both ends of the first conductive terminal 31. The first terminal end 311 of the first conductive terminal 31 is connected to the first lead electrode 21, and the second terminal portion 312 of the first conductive terminal 31 is used to connect to the detection circuit. The first terminal end 311 of the first conductive terminal 31 has a first tip 313, and the second terminal portion 312 of the first conductive terminal 31 has a second tip 314. Along the extension direction Z of the conductive terminal 3, the first lead electrode 21 and the first terminal end 311 of the first conductive terminal 31 are on the same side as the second tip 314 of the first conductive terminal 31. The second conductive terminal 32 has a first terminal end 321 and a second terminal portion 322. Along the extension direction Z of the conductive terminal 3, the first terminal end 321 and the second terminal portion 322 of the second conductive terminal 32 are located at both ends of the second conductive terminal 32. The first terminal end 321 of the second conductive terminal 32 is connected to the second lead electrode 22, and the second terminal portion 322 of the second conductive terminal 32 is used to connect to the detection circuit. The first terminal end 321 of the second conductive terminal 32 has a first tip 323, and the second terminal portion 322 of the second conductive terminal 32 has a second tip 324. Along the extension direction Z of the conductive terminal 3, the second lead electrode 22 and the first terminal end 321 of the second conductive terminal 32 are on the same side as the second tip 324 of the second conductive terminal 32. In some embodiments of this application, the height direction of the detection assembly 100 is aligned with the extension direction Z of the conductive terminal 3.

In some embodiments, the first tip 313 of the first conductive terminal 31, the second tip 314 of the first conductive terminal 31, the first tip 323 of the second conductive terminal 32, and the second tip 324 of the second conductive terminal 32 can each be an end face, for example, a flat surface, a curved surface, or a rough surface. Alternatively, when the first terminal ends 311, 321, or the second terminal portions 312, 322 are conical, the first tips 313, 323 are point-shaped, or the second tips 314, 324 are point-shaped.

To reduce the deformation of the lead electrode 2 under external forces, this application designs the relative position of the lead electrode 2 and the conductive terminal 3. The following explanation uses the relative position of the first lead electrode 21 and the first conductive terminal 31 as an example.

The first lead electrode 21 includes a first connection portion 211 and a first extension portion 212. The first connection portion 211 is connected to the first terminal end 311 of the first conductive terminal 31, and the first extension portion 212 extends from the first connection portion 211 in a direction away from the first conductive terminal 31, as shown in Figures 3~6.

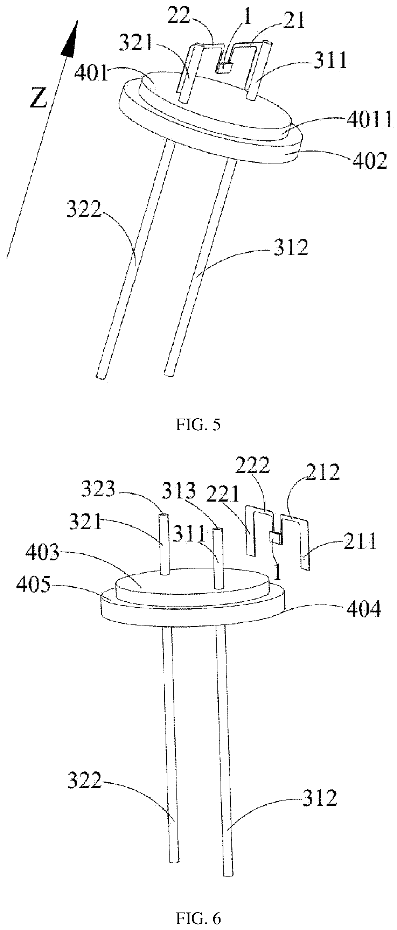

In some embodiments, along the extension direction Z of the conductive terminal 3, the first connection portion 211 and the second terminal portion 312 of the first conductive terminal 31 are on the same side as the first tip 313 of the first conductive terminal 31, as shown in Figures 3~6, or Figure 21, or Figures 9∼11. The first terminal end 311 has a side surface 315, and the first connection portion 211 is connected to the side surface 315, as shown in Figures 4 and 6. Since the diameter of the first conductive terminal 31 is small, while the side surface 315 of the first conductive terminal 31 has a larger area, connecting the first connection portion 211 to the side surface 315 of the first conductive terminal 31 helps reduce the difficulty of connecting the first lead electrode 21 to the first conductive terminal 31. As such, in the extension direction of the conductive terminal 3, the first connection portion 211 neither protrudes beyond the first tip 313 nor the second tip 314, allowing the first conductive terminal 31 to block external forces for the first connection portion 211, thus reducing the deformation of the first connection portion 211 under external forces.

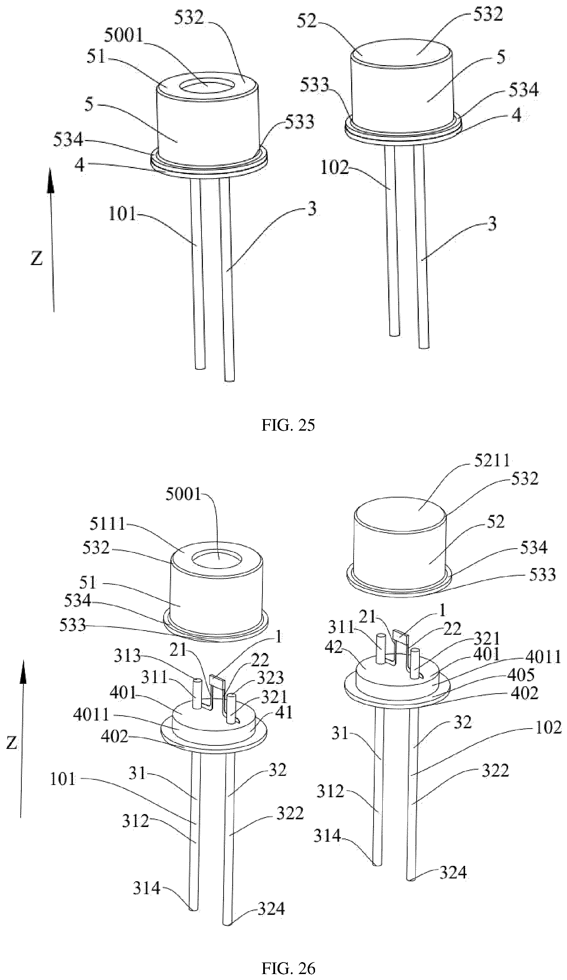

At least part of the first extension portion 212 is on the same side as the second terminal portion 312 of the first conductive terminal 31 relative to the first tip 313 of the first conductive terminal 31, as shown in Figure 26. This reduces the height of the portion of the first lead electrode 21 protruding from the first tip 313 of the first conductive terminal 31 in the height direction of the detection assembly 100, that is, it reduces the height of the first extension portion 212, thereby reducing the height of the detection assembly 100.

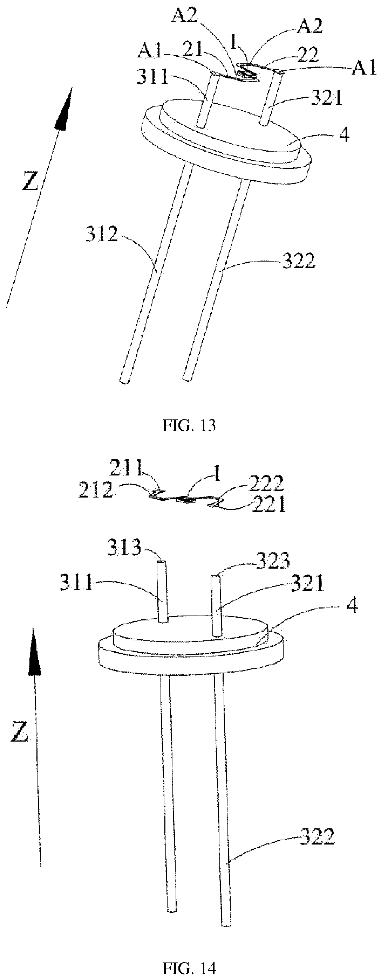

In other embodiments, along the extension direction Z of the conductive terminal 3, the first connection portion 211 and the second terminal portion 312 of the first conductive terminal 31 are on different sides of the first tip 313 of the first conductive terminal 31, and the first connection portion 211 is in a plane perpendicular to the extension direction of the conductive terminal, as shown in Figures 12∼15, or Figures 17 and 18. The first connection portion 211 is connected to the first tip 313, slightly higher than the first tip 313, and approximately flush with the first tip 313. In this case, along the extension direction Z of the conductive terminal, the height difference between the top A1 of the first connection portion 211 and the first tip 313 is approximately equal to the thickness of the first connection portion 211 in the direction perpendicular to the height direction of the detection assembly 100, as shown in Figures 13 and 14. This also helps reduce the deformation of the first connection portion 211 under external forces.

In some embodiments, along the extension direction Z of the conductive terminal 3, the first extension portion 212 and the second terminal portion 312 of the first conductive terminal 31 are on the same side as the first tip 313 of the first conductive terminal 31, as shown in Figures 3~6, or Figures 9∼11. As such, in the extension direction of the conductive terminal, the first extension portion 212 neither protrudes beyond the first tip 313 nor the second tip 314. When the detection assembly 100 collides with external substances, the first conductive terminal 31 can block part of the external forces for the first extension portion 212, thereby reducing the deformation of the first extension portion 212.

In other embodiments, the first extension portion 212 and the second terminal portion 312 of the first conductive terminal 31 are on different sides of the first tip 313 of the first conductive terminal 31, and the first extension portion 212 is in a plane perpendicular to the extension direction of the conductive terminal, as shown in Figures 12∼15, or Figures 17 and 18. The first extension portion 212 is slightly higher than the first tip 313, and approximately flush with the first tip 313. In this case, along the extension direction of the conductive terminal, the height difference between the top A2 of the first extension portion 212 and the first tip 313 is approximately equal to the thickness of the first extension portion 212 in the direction perpendicular to the height direction of the detection assembly 100. This also helps reduce the deformation of the first extension portion 212 under external forces.

In this application, along the extension direction Z of the conductive terminal 3, whether the first lead electrode 21 is not higher than the first tip 313 of the first conductive terminal 31, or the first lead electrode 21 is slightly higher than the first tip 313 of the first conductive terminal 31, both can reduce the deformation of the first lead electrode 21 under external forces. Moreover, it can also reduce the overall height of the detection assembly 100, which is conducive to the miniaturization of the detection assembly 100.

In some embodiments, along the extension direction Z of the conductive terminal 3, the first connection portion 211 and the first extension portion 212 are on different sides of the first tip 313 of the first conductive terminal 31. For example, as shown in Figure 19, the first connection portion 211 is connected to the first tip 313 of the first conductive terminal 31, and along the extension direction Z of the conductive terminal 3, the first connection portion 211 and the second terminal portion 312 of the first conductive terminal 31 are on different sides of the first tip 313 of the first conductive terminal 31, and the first connection portion 211 is in a plane perpendicular to the extension direction of the conductive terminal; along the extension direction Z of the conductive terminal 3, the first extension portion 212 and the second terminal portion 312 of the first conductive terminal 31 are on the same side as the first tip 313 of the first conductive terminal 31. As another example, as shown in Figure 20, along the extension direction Z of the conductive terminal 3, the first connection portion 211 and the second terminal portion 312 of the first conductive terminal 31 are on the same side as the first tip 313 of the first conductive terminal 31, while the first extension portion 212 and the second terminal portion 312 of the first conductive terminal 31 are on different sides of the first tip 313 of the first conductive terminal 31, and the first extension portion 212 is in a plane perpendicular to the extension direction of the conductive terminal.

In other embodiments, along the extension direction Z of the conductive terminal 3, the first connection portion 211 and the first extension portion 212 are on the same side as the first tip 313 of the first conductive terminal 31, as shown in Figures 3~6, or Figures 9∼11. Along the extension direction Z of the conductive terminal 3, the first connection portion 211 and the first extension portion 212 of the first lead electrode 21 are on the same side as the second terminal portion 312 of the first conductive terminal 31 relative to the first tip 313 of the first conductive terminal 31. For example, as shown in Figures 12~15, or Figures 17~19, along the extension direction Z of the conductive terminal 3, the first connection portion 211 and the first extension portion 212 of the first lead electrode 21 are on different sides of the first tip 313 of the first conductive terminal 31 relative to the second terminal portion 312 of the first conductive terminal 31, and the first lead electrode 21 is in a plane perpendicular to the extension direction of the conductive terminal. Compared to the design where the first connection portion 211 and the first extension portion 212 are on different sides of the first tip 313 of the first conductive terminal 31, having the first connection portion 211 and the first extension portion 212 on the same side as the first tip 313 of the first conductive terminal 31 can reduce the bending or twisting of the first lead electrode 21, thereby reducing the impact of internal stress on signal transmission in the first lead electrode 21.

Similarly, the second lead electrode 22 includes a second connection portion 221 and a second extension portion 222. The second connection portion 221 is connected to the first terminal end 321 of the second conductive terminal 32, and the second extension portion 222 extends from the second connection portion 221 in a direction away from the first terminal end 321 of the second conductive terminal 32, as shown in Figures 3~6. The relative position of the second lead electrode 22 and the second conductive terminal 32 is set up in the same way as the relative position of the first lead electrode 21 and the first conductive terminal 31 in this application, so it will not be repeated here.

In some embodiments, the first extension portion 212 is on the side of the first conductive terminal 31 facing the second conductive terminal 32, and the second extension portion 222 is on the side of the second conductive terminal 32 facing the first conductive terminal 31. For example, as shown in Figures 3~6, or Figure 21, or Figures 9~11, in the direction of the line connecting the center of the first conductive terminal 31 and the center of the second conductive terminal 32, the first extension portion 212 and the second conductive terminal 32 are on the same side of the first conductive terminal 31, and the second extension portion 222 and the first conductive terminal 31 are on the same side of the second conductive terminal 32. In other words, the first extension portion 212 and the second extension portion 222 are between the first conductive terminal 31 and the second conductive terminal 32. This allows the first conductive terminal 31 and the second conductive terminal 32 to block external forces for the first extension portion 212 and the second extension portion 222, reducing the deformation of the first extension portion 212 and the second extension portion 222 under external forces.

The lead electrode 2 can be in the form of a thin film, sheet, or wire. Bending of the lead electrode 2 will cause an increase in internal stress, thereby affecting the transmission of electrical signals within the lead electrode 2. Therefore, in some embodiments, the lead electrode 2 is positioned in a plane that is parallel to the extension direction of the conductive terminal, as shown in Figures 3∼6; or, the plane is perpendicular to the extension direction of the conductive terminal, as shown in Figures 12∼15. This can reduce the bending of the lead electrode 2. Of course, in practice, due to the self-weight of the detection element or lead electrode, or other reasons, the lead electrode 2 may slightly deviate from the plane or not be completely within the plane. In this application, such situations are still considered as the lead electrode 2 being or approximately being in the plane.

In some embodiments, the first lead electrode 21 is in the form of a thin sheet, and the second lead electrode 22 is in the form of a thin sheet. For example, both the first lead electrode 21 and the second lead electrode 22 are thin sheet electrodes prepared by PVD method, with a thickness at the micron or millimeter level. In some embodiments, when the first lead electrode 21 is connected to the first conductive terminal 31, the second lead electrode 22 is connected to the second conductive terminal 32, and both the first lead electrode 21 and the second lead electrode 22 are connected to the same detection element 1, the first extension portion 212 is closer to the second conductive terminal 32 relative to the first connection portion 211. The second extension portion 222 is closer to the first conductive terminal 31 relative to the second connection portion 221, as shown in Figure 21.

In some embodiments, the first lead electrode 21 is flush with the second lead electrode 22. That is, the first lead electrode 21 and the second lead electrode 22 are in the same plane, which can be perpendicular or parallel to the extension direction of the conductive terminal. This can reduce the mutual influence between the first lead electrode 21 and the second lead electrode 22. Since both the first lead electrode 21 and the second lead electrode 22 are connected to the detection element 1, if the first lead electrode 21 and the second lead electrode 22 are not flush, at least one of the first lead electrode 21 and the second lead electrode 22 needs to bend. As described, bending of the lead electrode will cause internal stress, affecting the transmission of electrical signals. Therefore, setting the first lead electrode 21 and the second lead electrode 22 to be flush can reduce the generation of internal stress in the lead electrodes. Of course, in practice, due to the self-weight of the detection element or lead electrode, or other reasons, the first lead electrode 21 and the second lead electrode 22 may slightly deviate from the "flush" position. In this application, such situations are still considered as the first lead electrode 21 and the second lead electrode 22 being flush or substantially flush.

As described above, in some embodiments, the first lead electrode 21 is positioned in a plane parallel to the extension direction of the conductive terminal 3, and the first lead electrode 21 is connected to the side surface 315 of the first conductive terminal 31, as shown in Figures 3~6. The first connection portion 211 has a first connecting end 2111 and a second connecting end 2112, which are located at both ends of the first connection portion 211 along the height direction or width direction of the detection assembly 100. The first connecting end 2111 of the first connection portion 211 is closer to the first tip 313 of the first conductive terminal 31 relative to the second connecting end 2112 of the first connection portion 211. The first connection portion 211 has a first connection point 2113 that connects to the first extension portion 212. The first connection point 2113 can be located at the first connecting end 2111, as shown in Figures 3~6; or, the first connection point 2113 can be located at the second connecting end 2112, as shown in Figures 9∼11, or as shown in Figure 22; or, the first connection point 2113 can also be located at any position between the first connecting end 2111 and the second connecting end 2112. Placing the first connection point 2113 at the first connecting end 2111 or the second connecting end 2112 can reduce the waste of manufacturing materials for the first lead electrode 21.

Similarly, for the second lead electrode 22, it is positioned in a plane parallel to the extension direction of the conductive terminal, and the second lead electrode 22 is connected to the side surface 325 of the second conductive terminal 32, as shown in Figures 3~6. The second connection portion 221 has a first connecting end 2211 and a second connecting end 2212, which are located at both ends of the second connection portion 221 along the height direction or width direction of the detection assembly 100. The first connecting end 2211 of the second connection portion 221 is closer to the first tip 323 of the second conductive terminal 32 relative to the second connecting end 2212 of the second connection portion 221. The second connection portion 221 has a second connection point 2213 that connects to the second extension portion 222. The second connection point 2213 can be located at the first connecting end 2211 of the second connection portion 221, as shown in Figures 3~6; or, the second connection point 2213 can be located at the second connecting end 2212, as shown in Figures 9∼11, or as shown in Figure 22; or, the second connection point 2213 can also be located at any position between the first connecting end 2211 and the second connecting end 2212.

In some embodiments, the first connection portion 211 and the second connection portion 221 have the same shape, structure, and dimensions. In some embodiments, the first connection portion 211 and the second connection portion 221 are symmetrically arranged. For example, as shown in Figures 3, 7, and 8. Both the first connection portion 211 and the second connection portion 221 are thin sheet-like structures, both are strip-shaped, and in Figures 12~15, both the first connection portion 211 and the second connection portion 221 are circular. In Figures 3~8, or in Figures 9~11, or Figures 17 and 18, the first connection portion 211 and the second connection portion 221 are arranged with axial symmetry; in Figures 12~15, the first connection portion 211 and the second connection portion 221 are arranged with central symmetry.

In related technology, the lead electrode 2 and the conductive terminal 3 are connected by spot welding. The reliability of spot welding connections is poor, and the lead electrode 2 and the conductive terminal 3 are prone to separation at the connection point, causing connection failure.

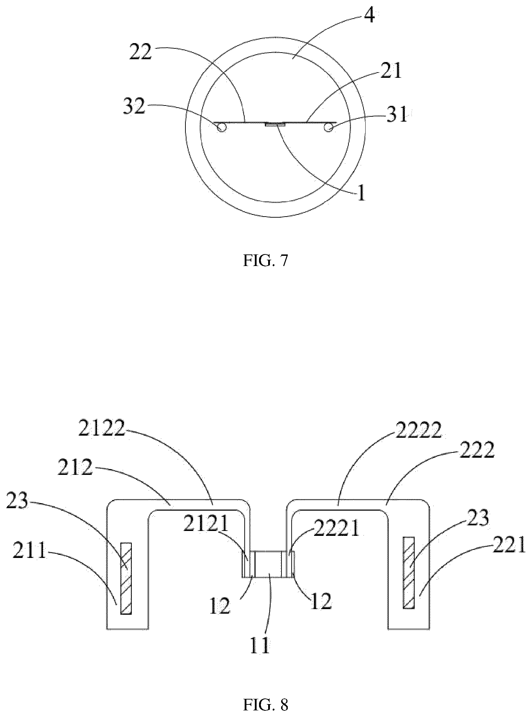

In some embodiments, the connection portion includes a connection area 23 for connecting with the conductive terminal 3, and the connection area 23 is in the form of a line or a surface. Taking the connection between the first lead electrode 21 and the first conductive terminal 31 as an example. When the connection area 23 of the first connection portion 211 is in the form of a line, as shown in Figure 8, the connection between the first connection portion 211 and the first conductive terminal 31 is a line connection; when the connection area 23 of the first connection portion 211 is in the form of a surface, the connection between the first connection portion 211 and the first conductive terminal 31 is a surface connection. Similarly, when the connection area 23 of the second connection portion 221 is in the form of a line, as shown in Figure 8, the connection between the second connection portion 221 and the second conductive terminal 32 is a line connection; when the connection area 23 of the second connection portion 221 is in the form of a surface, the connection between the second connection portion 221 and the second conductive terminal 32 is a surface connection.

To determine the shape of the connection area, the lead electrode 2 can be separated from the conductive terminal 3. If a line-shaped connection mark is left on the lead electrode 2 or the conductive terminal 3 after separation, it indicates that the connection between the lead electrode 2 and the conductive terminal 3 is line-shaped; if a surface-shaped connection mark is left on the lead electrode 2 or the conductive terminal 3 after separation, it indicates that the connection between the lead electrode 2 and the conductive terminal 3 is surface-shaped.

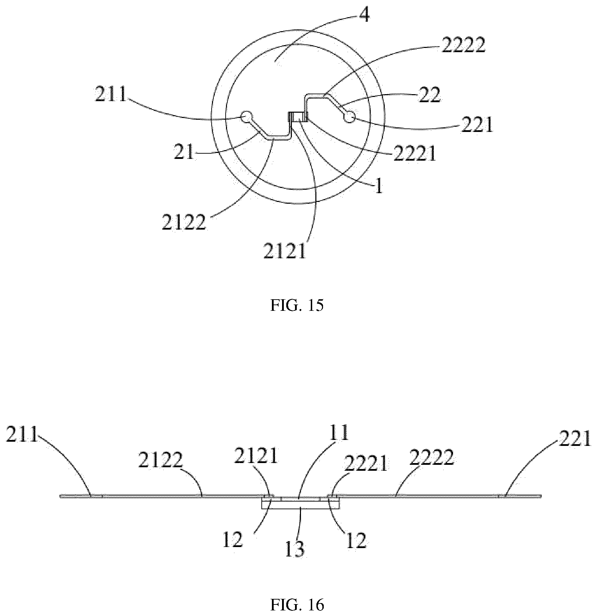

To achieve signal transmission, the lead electrode 2 not only needs to be connected to the conductive terminal 3 but also to the detection element 1. The detection element 1 includes a detection part 11 and at least one pair of electrode parts 12. The detection part 11 is electrically connected to the electrode part 12, and the electrode part 12 is electrically connected to the lead electrode 2, as shown in Figure 16. The detection part 11 can generate electrical signals under the influence of the substance or parameter being detected, for example, generating electrical signals when the gas concentration changes. The electrical signals are transmitted through the electrode part 12 to the lead electrode 2, and then through the conductive terminal 3 to the detection circuit.

In some embodiments, the detection element 1 includes an insulating substrate 13, the detection part 11 is a thermosensitive resistor material coated on the surface of the insulating substrate 13, and the electrode part 12 is a conductive layer coated on the surface of the insulating substrate 13, as shown in Figure 16. When changes in gas concentration cause changes in the temperature of the environment to which the thermosensitive resistor material is exposed, the resistance of the thermosensitive resistor material changes, thereby generating electrical signals. The electrical signals are transmitted to the detection circuit through the electrode part 12, the lead electrode 2, and the conductive terminal 3. In some embodiments, the plane in which the first lead electrode 21 and the second lead electrode 22 are located is parallel to at least part of the surface of the detection element 1. For example, it is parallel to the electrode part of the detection element. This can reduce the deformation of the lead electrode caused by its connection to the detection element.

In some embodiments, the plane in which the lead electrode 2 is located is parallel to the insulating substrate 13. This can reduce the bending of the lead electrode 2 caused by its connection to the detection element 1, as shown in Figure 16.

In some embodiments, along the extension direction Z of the conductive terminal 3, the detection part 11 is closer to the first tip 313 of the first conductive terminal or the first tip 323 of the second conductive terminal relative to the insulating substrate 13, as shown in Figures 12 and 16. This allows the detection part to be fully exposed to the environment.

In other embodiments, the detection element 1 includes a detection substrate and internal electrodes set within the detection substrate. The detection substrate is a ceramic substrate that includes thermosensitive resistor material. When changes in gas concentration cause changes in the temperature of the environment to which the detection substrate is exposed, the resistance of the detection substrate 14 changes, thereby generating electrical signals. The electrical signals are transmitted to the detection circuit through the internal electrodes, lead electrodes 2, and conductive terminals 3.

In the detection element 1, the electrode parts 12 are symmetrically arranged relative to the axis of the detection element 1, as shown in Figure 16. To facilitate the connection of the lead electrodes 2 to the detection element 1, a pair of lead electrodes 2 is also symmetrically arranged.

In some embodiments, the first extension portion 212 includes interconnected first connection segment 2121 and second connection segment 2122, where the first connection segment 2121 is connected to the electrode part 12 of the detection element 1, and the second connection segment 2122 extends from the first connection segment 2121 in a direction away from the detection element 1, with the second connection segment 2122 connected to the first connection portion 211; the second extension portion 222 includes interconnected third connection segment 2221 and fourth connection segment 2222, where the third connection segment 2221 is connected to the electrode part 12 of the detection element 1, and the fourth connection segment 2222 extends from the third connection segment 2221 in a direction away from the detection element 1, with the fourth connection segment 2222 connected to the second connection portion 221; the first connection segment 2121 is parallel to the third connection segment 2221, as shown in Figures 8 and 16. This facilitates the connection of a pair of lead electrodes 2 to the detection element 1.

In some embodiments, at least part of the second connection segment 2122 is parallel to at least part of the fourth connection segment 2222. For example, as shown in Figure 8, the second connection segment 2122 and the fourth connection segment 2222 extend in the same direction away from the detection element 1; as shown in Figure 16, the second connection segment 2122 and the fourth connection segment 2222 extend in opposite directions away from the detection element 1.

In some embodiments, along the height direction of the detection assembly 100, the first connection segment 2121 and the second terminal end 312 of the first conductive terminal 31 are located on opposite sides of the first tip 313 of the first conductive terminal 31, while the second connection segment 2122 and the second terminal end 312 of the first conductive terminal 31 are located on the same side of the first tip 313 of the first conductive terminal 31, as shown in Figure 22.

In some embodiments, at least a portion of the first connection segment 2121 and the second terminal end 312 of the first conductive terminal 31 are located on the same side of the first tip 313 of the first conductive terminal 31, and the second connection segment 2122 and the second terminal end 312 of the first conductive terminal 31 are located on the same side of the first tip 313 of the first conductive terminal 31. In some embodiments, the first extension portion 212 and the second terminal end 312 of the first conductive terminal 31 are located on the same side of the first tip 313 of the first conductive terminal 31. In other words, the first lead electrode 21 is not protruding from the first conductive terminal 31. In some embodiments, the axis of the second connection segment 2122 is a curve.

In some embodiments, along the height direction of the detection assembly 100, the third connection segment 2221 and the second terminal end 322 of the second conductive terminal 32 are located on opposite sides of the first tip 323 of the second conductive terminal 32, and the fourth connection segment 2222 and the second terminal end 322 of the second conductive terminal 32 are located on the same side of the first tip 323 of the second conductive terminal 32. Alternatively, at least a portion of the third connection segment 2221 and the second terminal end 322 of the second conductive terminal 32 are located on the same side of the first tip 323 of the second conductive terminal 32, and the fourth connection segment 2222 and the second terminal end 322 of the second conductive terminal 32 are located on the same side of the first tip 323 of the second conductive terminal 32. In other words, the second lead electrode 22 is not protruding from the second conductive terminal 32. In some embodiments, the axis of the fourth connection segment 2222 is a curve.

In some embodiments, at least a portion of the axis of the first connection segment 2121 is parallel to the axis of the first conductive terminal 31, and at least a portion of the axis of the third connection segment 2221 is parallel to the axis of the second conductive terminal 32, as shown in Figure 26. In some embodiments, the first extension portion 212 is arranged to be axisymmetric or centrosymmetric relative to the second extension portion 222. For example, as shown in Figures 3-8, or in Figures 9-11, or in Figure 21, the first lead electrode 21 and the second lead electrode 22 are located in a plane parallel to the extension direction of the conductive terminal 3, with the first extension portion 212 arranged to be axisymmetric relative to the second extension portion 222, and the axis of the orthographic projection of the detection element 1 on this plane is the axis of symmetry for the first extension portion 212 and the second extension portion 222. As shown in Figures 12-15, for example, the first lead electrode 21 and the second lead electrode 22 are located in a plane perpendicular to the extension direction of the conductive terminal 3, with the first extension portion 212 arranged to be centrosymmetric relative to the second extension portion 222, and the center of the orthographic projection of the detection element 1 on this plane is the center of symmetry for the first extension portion 212 and the second extension portion 222.

In some embodiments, the first lead electrode 21 is axi- or centro- symmetric relative to the second electrode 22. For example, as shown in Figures 12-15, the first lead electrode 21 and the second lead electrode 22 are located in a plane perpendicular to the extension direction of the conductive terminal, with the first lead electrode 21 arranged to be centrosymmetric relative to the second lead electrode 22, and the center of the orthographic projection of the detection element 1 on this plane is the center of symmetry for the first lead electrode 21 and the second lead electrode 22. As shown in Figures 3-8, or in Figures 9-11, or in Figure 21, for example, the first lead electrode 21 and the second lead electrode 22 are located in a plane parallel to the extension direction of the conductive terminal, with the first lead electrode 21 arranged to be axisymmetric relative to the second lead electrode 22, and the axis of the orthographic projection of the detection element 1 on this plane is the axis of symmetry for the first lead electrode 21 and the second lead electrode 22. The symmetric structure of the first lead electrode 21 and the second lead electrode 22 can facilitate the transmission of electrical signals from the detection element 1.

As described in this application, along the extension direction Z of the conductive terminal 3, the lead electrode 2 is not higher than or slightly higher than the first tip 313 of the first conductive terminal 31. The detection element 1 is electrically connected to the lead electrode 2, therefore, the detection element 1 is also not higher than or slightly higher than the first tip 313 of the first conductive terminal 31.

Below, we will use the relative position of the detection element 1 and the first conductive terminal 31 as an example for explanation. In some embodiments, the detection element 1 has a first side 111 and a second side 112, which are located on different sides of the detection element 1 along the extension direction Z of the conductive terminal 3. Along the extension direction of the conductive terminal, both the first side 111 and the second side 112 are on the same side of the first tip 313 of the first conductive terminal 31 as the second terminal end 312 of the first conductive terminal 31, as shown in Figure 12 or Figure 22. Alternatively, the first side 111 and the second terminal end 312 of the first conductive terminal 31 are on different sides of the first tip 313, the second side 112 and the second terminal end 312 of the first conductive terminal 31 are on different sides of the first tip 313 of the first conductive terminal 31, along the extension direction of the conductive terminal, the first side 111 is farther from the first tip 313 of the first conductive terminal 31 relative to the second side 112, and the second side 112 is flush with the first tip 313 of the first conductive terminal 31. In this case, along the extension direction Z of the conductive terminal 3, the height difference between the first side 111 of the detection element 1 and the first tip 313 of the first conductive terminal 31 is approximately equal to the dimension of the detection element 1 in the extension direction of the conductive terminal 3. Alternatively, the first side 111 and the second side 112 are on different sides of the first tip 313 of the first conductive terminal 31, as shown in Figure 20.

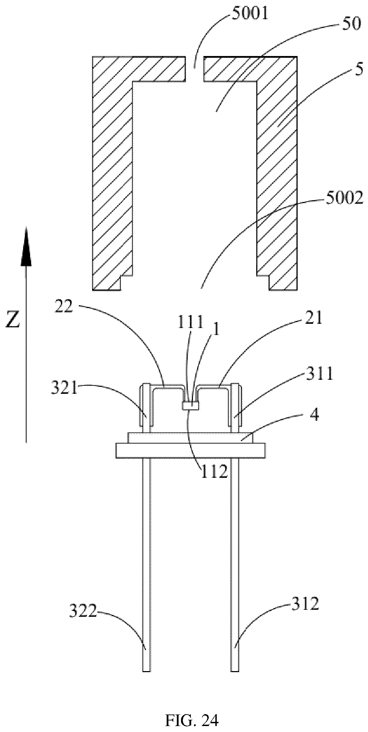

Similarly, the relative position of the detection element 1 and the second conductive terminal 32 can be arranged. In some embodiments, along the extension direction Z of the conductive terminal 3, both the first side 111 and the second side 112 are on the same side of the first tip 323 of the second conductive terminal 32 as the second terminal end 322 of the second conductive terminal 32, as shown in Figure 12 or Figure 24. Alternatively, the first side 111 and the second terminal end 322 of the second conductive terminal 32 are on different sides of the first tip 313, the second side 112 and the second terminal end 322 of the second conductive terminal 32 are on different sides of the first tip 313 of the first conductive terminal 31, along the extension direction Z of the conductive terminal 3, the first side 111 is farther from the first tip 323 of the second conductive terminal 32 relative to the second side 112, and the second side 112 is flush with the first tip 323 of the second conductive terminal 32. In this case, along the extension direction Z of the conductive terminal 3, the height difference between the second side 112 of the detection element 1 and the first tip 323 of the second conductive terminal 32 is approximately equal to the dimension of the detection element 1 in the extension direction of the conductive terminal. Alternatively, the first side 111 and the second side 112 are on different sides of the first tip 323 of the second conductive terminal 32, as shown in Figure 20.

In this way, the first conductive terminal 31 and the second conductive terminal 32 can be used to provide protection for the detection element 1, which can reduce the effect of external forces on the detection element. For example, during the assembly process of the detection assembly 100 and the housing 5, it reduces the collision between the detection element and the inner wall of the housing 5.

In some embodiments, the detection assembly 100 also includes a base 4, with the conductive terminal 3 fixedly connected to the base 4 and passing through the base 4, as shown in Figures 5 and 13. Along the extension direction Z of the conductive terminal 3, the first terminal end 311 and the second terminal end 312 of the first conductive terminal 31 are located on different sides of the base 4, and the first terminal end 321 and the second terminal end 322 of the second conductive terminal 32 are located on different sides of the base 4.

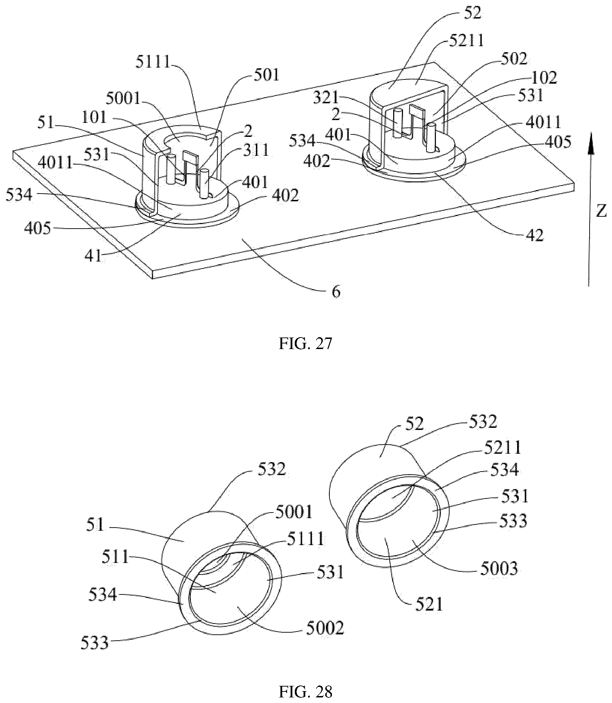

The base 4 includes a first support platform 401 and a second support platform 402, with the first support platform 401 connected to the second support platform 402, and the conductive terminal 3 passing through both the first support platform 401 and the second support platform 402. Along the height direction of the detection assembly 100, the projection of the first support platform 401 on the second support platform 402 is within the outer contour of the second support platform 402. For example, as shown in Figures 26 and 27, both the first support platform 401 and the second support platform 402 are cylindrical, with the outer diameter of the first support platform 401 being smaller than the outer diameter of the second support platform 402, and the central axis of the first support platform 401 coinciding with the central axis of the second support platform 402. Along the height direction of the detection assembly 100, the first support platform 401 has a first end face 403, which is a flat surface; the second support platform 402 has a second end face 404 and a third end face 405, with the third end face 405 being closer to the first end face 403 relative to the second end face 404, and both the second end face 404 and the third end face 405 being circular flat surfaces. The base 4 has a stepped shape. The radius of the first end face 403 is smaller than the radius of the second end face 404.

Along the height direction of the detection assembly 100, the second end face 404 is farther from the first terminal end 311 of the first conductive terminal 31 (or the first terminal end 321 of the second conductive terminal 32) relative to the first end face 403, and the first end face 403 is closer to the first lead electrode 21 and the second lead electrode 22 relative to the second end face 404.

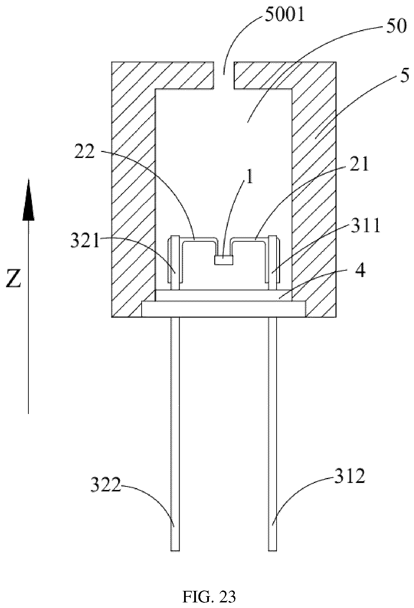

The second aspect of this application provides a sensor, as shown in Figure 23, which includes the detection assembly 100 as described above, a housing 5, and a circuit board 6. The housing 5 is connected to the circuit board 6, and the detection assembly 100 is electrically connected to the circuit board 6. The conductive terminal 3 of the detection assembly 100 is electrically connected to the circuit board 6, and the conductive terminal 3 is fixedly connected to the circuit board 6.

By adopting the detection assembly as described above, not only can it reduce deformation caused by collision between the detection assembly and the inner wall of the housing during the assembly process, but it can also reduce the overall height of the sensor, which is conducive to the miniaturization of the overall sensor.

The housing 5 has a chamber 50, which has a first opening 5001. The chamber 50 is in fluid communication with the external environment of the sensor through the first opening 5001. Both the detection element 1 and the lead electrode 2 are located within the cavity 50. The first terminal end 311 of the first conductive terminal 31 is located within the chamber 50, while the second terminal end 312 of the first conductive terminal 31 is located outside the cavity 50; the first terminal end 321 of the second conductive terminal 32 is located within the chamber 50, while the second terminal end 322 of the second conductive terminal 32 is located outside the chamber 50.

During the use of the sensor, the first opening 5001 is in communication with the external environment of the sensor, allowing the gas to be detected to enter the chamber 50 through the first opening 5001, causing a change in the resistance of the detection part 11 of the detection element 1 and generating an electrical signal. The electrical signal is transmitted to the detection circuit through the lead electrode 2 and the conductive terminal 3, thereby achieving the detection of relevant parameters.

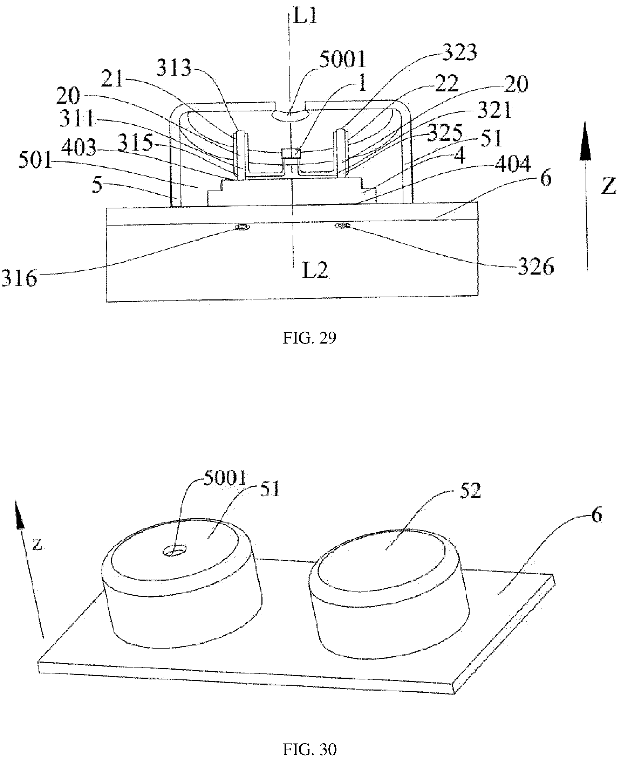

In some embodiments, the housing 5 is directly connected to the base 4, with the housing 5 and base 4 being fixedly connected or position-limited, as shown in Figures 25-27; alternatively, the housing 5 is directly connected to the circuit board 6, with the housing 5 and circuit board 6 being fixedly connected or position-limited, and the base 4 is located inside the housing 5, with at least a portion of the housing 5 having a gap between it and the base 4, as shown in Figures 29-32. In other words, the base 4 and housing 5 are separately fixedly connected to the circuit board 6. The sensor may experience displacement or rotation during transportation and use, and separately connecting the base 4 and housing 5 to the circuit board 6 can reduce damage to the sensor caused by collision or interference between the detection assembly 100, housing 5, and circuit board 6 due to displacement or rotation.

The separate connection of the base 4 and housing 5 to the circuit board 6 means that the base 4 is directly connected to the circuit board 6, and the housing 5 is directly connected to the circuit board 6, but the base 4 is not connected to the circuit board 6 through the housing 5, nor is the housing 5 connected to the circuit board 6 through the base 4.

In some embodiments, the fixed connection can be adhesive bonding or welding, where welding can be resistance welding, laser welding, ultrasonic welding, or other methods. The position-limited connection can be a detachable connection method, such as snap-fitting.

In some embodiments, the housing 5 has a peripheral wall 531, which is located around the chamber 50. The housing 5 has a third side 532 and a fourth side 533, with the third side 532 being farther from the circuit board 6 relative to the fourth side 533 along the thickness direction of the circuit board 6. In some embodiments, the fourth side 533 has a flanged part 534, which extends from the peripheral wall 531 in a direction away from the housing 5, with at least a portion of the flanged part 534 welded or adhesively bonded to the base 4; alternatively, at least a portion of the flanged part 534 is welded or adhesively bonded to the circuit board 6. The inclusion of the flanged part 534 can increase the connection area between the housing 5 and the base 4 or circuit board 6, improving connection reliability. In other embodiments, the housing 5 may not have a flanged part 534, with the outer end face of the housing 5 fixedly connected to the circuit board 6, as shown in Figures 29-32.

In some embodiments, the housing 5 is welded or adhesively bonded to the first support platform 401 and the second support platform 402 of the base 4. Specifically, at least a portion of the peripheral wall 531 of the housing 5 is connected to the outer peripheral wall 4011 of the first support platform 401, and at least a portion of the flanged part 534 of the housing 5 is connected to the third end face 405 of the second support platform 402. This can increase the connection reliability between the base 4 and the housing 5.

In some embodiments, the surface of the base 4 used for connecting to the circuit board 6 is flat, and the surface of the circuit board 6 used for connecting to the base 4 is also flat. This way, the connection between the base 4 and the circuit board 6 is achieved through a flat-to-flat connection, reducing connection difficulty and increasing connection area and reliability.

In some embodiments, the outer diameter of the base 4 is smaller than the inner diameter of the housing 5. This allows the housing 5 to cover the outer periphery of the base 4.

In some embodiments, the base 4 has a first central axis L1, and the housing 5 has a second central axis L2. The first central axis L1 coincides with the second central axis L2, as shown in Figure 29. This allows the detection assembly 100 to be positioned at the center of the housing 5.

In some embodiments, the sensor has a first cavity 501 and a second cavity 502. The first cavity 501 is in fluid communication with the external environment of the sensor and is an open cavity, while the second cavity 502 is a closed cavity. The detection assembly 100 includes a first detection assembly 101 and a second detection assembly 102. The first detection assembly 101 is at least partially located in the first cavity 501, and the second detection assembly 102 is at least partially located in the second cavity 502. The second detection assembly 102 serves as a reference component for the first detection assembly 101, as shown in Figure 27, used to correct the detection results of the first detection assembly 101 and improve detection accuracy. During detection, the gas to be detected enters the open cavity, changing the thermal conductivity of the gas in the open cavity, which in turn changes the resistance value of the thermistor in the first detection assembly 101. Correspondingly, the gas to be detected cannot enter the closed cavity to affect the resistance value of the thermistor in the second detection assembly 102.

In related technologies, since the housing of the first detection assembly 101 is connected to the housing of the second detection assembly 102, the heat/cold carried by the gas to be detected entering the open cavity can easily be conducted to the second detection assembly 102 through the housing, causing changes in the resistance value of the thermistor in the second detection assembly 102. This affects its correction of the detection results, thereby reducing detection accuracy.

To reduce the mutual influence between the first detection assembly 101 and the second detection assembly 102, this application designs the housing 5. The housing 5 includes a first housing 51 and a second housing 52. The first housing 51 is located around the first cavity 501, and the second housing 52 is located around the second cavity 502. The first housing 51 and the second housing 52 are separately arranged, with a gap between the first housing 51 and the second housing 52. In other words, the first housing 51 and the second housing 52 do not share a common wall, as shown in Figures 25 and 26. In some other embodiments, the separately arranged first housing 51 and second housing 52 can also be connected together by a connecting plate.

In some embodiments, the first housing 51 has a first chamber 511, at least a portion of which forms the first cavity 501. The first chamber 511 has a first opening 5001, which provides gas communication between the first cavity 501 and the external environment of the sensor. The second housing 52 has a second chamber 521, at least a portion of which forms the second cavity 502. The first housing 51 also has a second opening 5002, which is fully covered by the circuit board 6. Along the height direction of the first housing 51, the first opening 5001 and the second opening 5002 are located on opposite sides of the first housing 51.

The second housing 52 has a third opening 5003, with a wall surrounding the third opening 5003. The circuit board 6 fully covers the third opening 5003, and along the height direction of the sensor, the projection of the wall on the circuit board is within the outer contour of the circuit board 6.

In some embodiments, the first opening 5001 is located on the third side 532 of the first housing 51, and the second opening 5002 is located on the fourth side 533 of the first housing 51. For example, as shown in Figure 28, the first housing 51 has a first side wall 5111 located on the third side 532, with the first opening 5001 set in the first side wall 5111. The fourth side 533 of the first housing 51 is welded to the first base 41. Along the extension direction Z of the conductive terminal 3, the projection of the peripheral wall 531 surrounding the second opening 5002 of the first housing 51 on the first base 41 is within the outer contour of the first base 41. The third opening 5003 is located on the fourth side 533 of the second housing 52. The second housing 52 has a second side wall 5211 located on the third side 532 of the second housing 52. The fourth side 533 of the second housing 52 is welded to the second base 42, and the third side 532 of the second housing 52 is hermetically connected to the second base 42, forming a closed cavity with the second base 42, which is the closed second cavity 502. Along the height direction of the detection assembly, the projection of the peripheral wall 531 surrounding the third opening 5003 of the second housing 52 on the second base 42 is within the outer contour of the second base 42. In other words, the first base 41 fully covers the second opening 5002 of the first housing 51, and the second base 42 fully covers the third opening 5003 of the second housing 52.

In some embodiments, at least one of the first housing 51 and the second housing 52 has a flanged part 534.

The base 4 includes a first base 41 and a second base 42. The first housing 51 and the first base 41 are both located around the first cavity 501, with the first central axis L1 of the first base 41 passing through the first opening 5001. The second housing 52 and the second base 42 are both located around the second cavity 502, with the second housing 52 hermetically connected to the second base 42; alternatively, the circuit board 6 is located around the first cavity 501 and the second cavity 502, the first base 41 is located in the first cavity 501, the second base 42 is located in the second cavity 502, and the second housing 52 is hermetically connected to the circuit board 6.

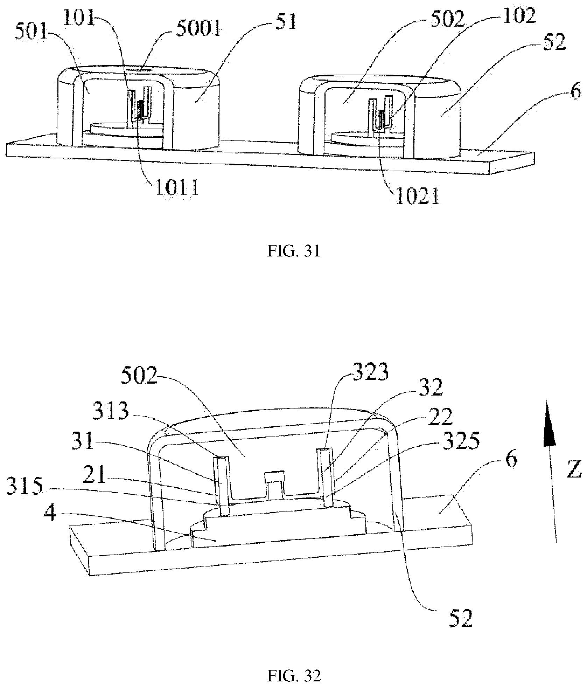

The first detection assembly 101 includes a first detection element 1011, and the second detection assembly 102 includes a second detection element 1021. For example, as shown in Figure 31, the first detection element 1011 is located in the first cavity 501, and the second detection element 1021 is located in the second cavity 502. Since the first housing 51 and the second housing 52 are separately arranged and do not share a common side wall, this can reduce heat transfer between the first detection assembly 101 and the second detection assembly 102.

To make the detection environments of the first detection assembly 101 and the second detection assembly 102 as similar as possible, the second detection assembly 102 can be arranged symmetrically to the first detection assembly 101.

In some embodiments, the height direction of the first detection assembly 101 is parallel or aligned with the height direction of the second detection assembly 102. In some embodiments, the axis of symmetry of the first detection assembly 101 is collinear with the axis of symmetry of the second detection assembly 102. In some embodiments, the axis of the first housing 51 is collinear with the axis of the second housing 52.

The first detection assembly 101 and the second detection assembly 102 each include at least one pair of conductive terminals 3 and at least one pair of lead electrodes 2. The at least one pair of conductive terminals 3 includes a first conductive terminal 31 and a second conductive terminal 32, and the at least one pair of lead electrodes 2 includes a first lead electrode 21 and a second lead electrode 22. For example, as shown in Figure 26, the first conductive terminal 31 is electrically connected to the first lead electrode 21, and the second conductive terminal 32 is electrically connected to the second lead electrode 22. The first detection element 1011 is electrically connected to the first conductive terminal 31 and the second conductive terminal 32. The second detection element 1021 is electrically connected to the first conductive terminal 31 and the second conductive terminal 32.

In some embodiments, the at least one pair of lead electrodes 21, 22 is arranged to be axisymmetric relative to the first central axis L1, the at least one pair of conductive terminals 31, 32 is arranged to be axisymmetric relative to the first central axis L1, and the detection element 1 is arranged to be axisymmetric relative to the first central axis L1.

During the detection process, gas enters the first cavity 501 through the first opening 5001 located in the first housing 51, causing changes in the electrical signal, such as resistance, of the detection element 1 of the first detection assembly 101. The second detection assembly 102 serves as a reference component for the first detection assembly 101, and needs to be able to exclude the influence of environmental factors other than contact with the gas to be detected on the detection results. Therefore, in an ideal state, the first detection assembly 101 and the second detection assembly 102 are in the same environment, meaning that apart from whether they are in contact with the gas to be detected, the environmental factors affecting the first detection assembly 101 and the second detection assembly 102 are roughly the same. For example, when external gas comes into contact with the first housing 51 and the second housing 52 respectively, the temperature change of the first housing 51 caused by the contact with external gas is approximately equal to the temperature change of the second housing 52 caused by the contact with external gas. As a result, the change in electrical signal of the first detection assembly 101 caused by heat transfer between the first housing 51 and the first detection assembly 101 is approximately equal to the change in electrical signal of the second detection assembly 102 caused by heat transfer between the second housing 52 and the second detection assembly 102.

In simple terms, for the case where the detection elements of both the first detection assembly 101 and the second detection assembly 102 are thermistors, assume that the change in resistance of the thermistor of the first detection assembly 101 caused by the gas to be detected entering the first cavity 501 is R1, and the change in resistance of the thermistor of the first detection assembly 101 caused by other factors (for example, heat conduction between the gas flowing outside the first housing 51 and the first detection assembly 101 through the first housing 51, etc.) is R2. Thus, the detected change in resistance of the thermistor of the first detection assembly 101 is Rx, where Rx = R1 + R2. Ideally, the second detection assembly 102 is in the same environment as the first detection assembly 101, so the change in resistance of the thermistor of the second detection assembly 102 is Ry = R2. In other words, the difference between the detection environment of the first detection assembly 101 and that of the second detection assembly 102 is that the thermistor of the first detection assembly 101 is in contact with the gas to be detected, while the thermistor of the second detection assembly 102 is not. Therefore, the change in resistance caused by factors other than "contact with the gas to be detected" should be reflected in the detection result Ry of the second detection assembly 102. When correcting the detection results, Rx - Ry = R1, thus obtaining the change in resistance of the thermistor of the first detection assembly 101 solely due to contact with the gas to be detected, and then calculating the relevant parameters of the gas to be detected (such as gas concentration), improving detection accuracy. Ideally, when the concentration of the gas to be detected is 0, Rx = Ry. However, in reality, due to the different positions of the first detection assembly 101 and the second detection assembly 102, there are still differences in their environments, resulting in Ry ≠ R2. For example, the flow rate of gas to the first housing 51 may not be equal to the flow rate of gas to the second housing 52, or the temperature of the gas flowing to the first housing 51 may be different from that flowing to the second housing 52, and so on. To improve detection accuracy, it is necessary to make the environments of the first detection assembly 101 and the second detection assembly 102 as similar as possible, so that Ry approaches R2 infinitely. To achieve this, the first detection assembly 101 and the second detection assembly 102 have the same structure and dimensions, and the relative position of the first detection assembly 101 to the first housing 51 is also roughly consistent with the relative position of the second detection assembly 102 to the second housing 52.

In some embodiments, the circuit board 6 is plate-shaped. When the circuit board 6 is plate-shaped, the first housing 51 and the second housing 52 are arranged side by side on the same side of the circuit board 6. For example, as shown in Figures 30 and 31, both the first housing 51 and the second housing 52 are cylindrical, protruding from the same side of the circuit board 6. The axis of the first housing 51 and the axis of the second housing 52 are both perpendicular to the circuit board 6, with the axis of the first housing 51 passing through the first opening 5001 of the first housing 51.

The third aspect of this application provides a manufacturing method for a sensor, including the following steps:
S1, provide a detection assembly 100, housing 5, and circuit board 6. The detection assembly 100 includes a detection element 1, conductive part 20, and base 4, as shown in Figure 29. The detection element 1 is connected to the conductive part 20, and the conductive part 20 is connected to the base 4;
S2, connect the detection assembly 100 to the circuit board 6;
S3, fit the housing 5 over at least part of the detection assembly 100, and connect the housing 5 to the circuit board 6.

In the manufacturing method for the sensor provided in this application, the detection assembly 100 and housing 5 are separately connected to the circuit board 6. Compared to the manufacturing method of first connecting the base and housing to form a detection probe and then connecting the detection probe to the circuit board, this method reduces the assembly difficulty between the base 4 and housing 5, thereby reducing the manufacturing difficulty of the sensor.

In some embodiments, the connection in step S2 is both a fixed connection and an electrical connection. In other words, step S2 not only achieves a fixed connection between the detection assembly 100 and the circuit board 6 but also achieves an electrical connection between them.

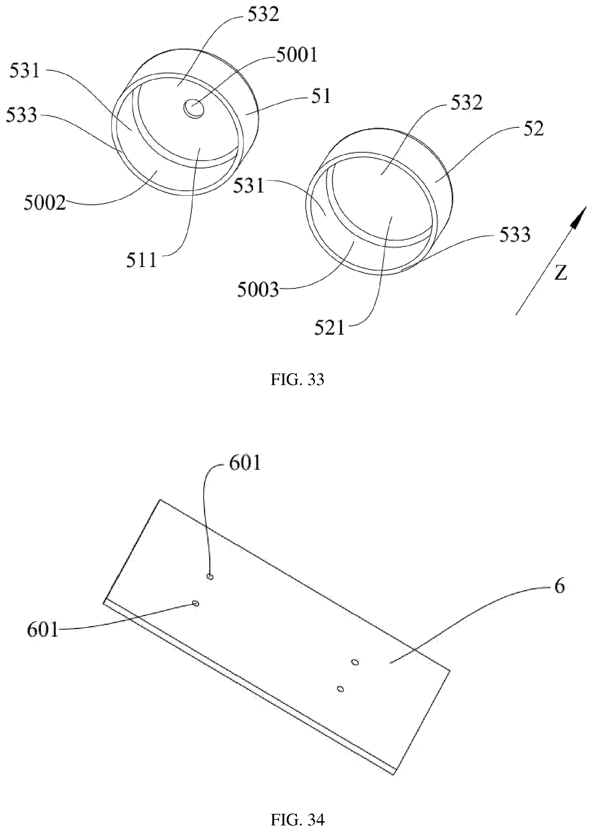

In some embodiments, the detection element 1 of the detection assembly 100 is electrically and fixedly connected to the conductive part 20, the conductive part 20 is fixedly connected to the base 4, and the conductive part 20 is electrically connected to the circuit board 6. Specifically, the circuit board 6 is provided with through-holes 601 for installing the conductive part 20, as shown in Figure 34. In some embodiments, step S2 of connecting the detection assembly 100 and the circuit board 6 includes: passing the conductive part 20 through the through-holes 601, filling the through-holes 601 with conductive material, and electrically and fixedly connecting the conductive part 20 and the circuit board 6 through the conductive material.

In some embodiments, after step S2 is completed, there is a gap between the base 4 and the circuit board 6. This reduces the impact of the detection assembly 100 on the heat dissipation of the circuit board 6.

In some embodiments, there is at least one pair of through-holes 601 set on the circuit board 6, and the conductive part 20 includes at least one pair of lead electrodes 21, 22 and at least one pair of conductive terminals 31, 32. The detection element 1 is connected to at least one pair of lead electrodes 21, 22, and at least one pair of lead electrodes 21, 22 is connected to at least one pair of conductive terminals 31, 32. At least one pair of conductive terminals 31, 32 passes through the base 4 and is fixedly connected to the base 4. The structures of the lead electrodes and conductive terminals, as well as the connection relationship between the lead electrodes and conductive terminals, are as described earlier.

In some embodiments, after step S2 of connecting the detection assembly 100 and the circuit board 6, there is an additional step: trimming the conductive terminals to remove the second terminal end of the conductive terminals. The first conductive terminal 31 includes a transition section 316, which is located within the through-hole 601 of the circuit board 6. One end of the transition section 316 is connected to the first terminal end 311 of the first conductive terminal 31, and the other end is connected to the second terminal end 312 of the first conductive terminal 31, as shown in Figure 29. After trimming the first conductive terminal 31, it only includes the first terminal end 311 located in the first cavity 501 and the transition section 316 located in the through-hole 601. Similarly, the second conductive terminal 32 includes a transition section 326, which is located within the through-hole 601 of the circuit board 6. One end of the transition section 326 is connected to the first terminal end 321 of the second conductive terminal 32, and the other end is connected to the second terminal end 322 of the second conductive terminal 32. After trimming the second conductive terminal 32, it only includes the first terminal end 321 located in the second cavity 502 and the transition section 326 located in the through-hole 601, as shown in Figure 29. In some embodiments, the first terminal end 311, transition section 316, and second terminal end 312 of the first conductive terminal 31 are coaxial, and the radius of the first conductive terminal 31 is constant; the first terminal end 321, transition section 326, and second terminal end 322 of the second conductive terminal 32 are coaxial, and the radius of the second conductive terminal 32 is constant.

In some embodiments, the housing 5 includes a first housing 51 and a second housing 52. Step S3 of connecting the housing 5 and the circuit board 6 includes the following steps:

S31, fixedly connect the first housing 51 and the circuit board 6, with at least part of the first chamber 511 forming the first cavity 501. The first housing 51 and the circuit board 6 are both located on the periphery of the first cavity 501, and the first opening 5001 provides gas communication between the first cavity 501 and the exterior of the sensor;

S32, fixedly connect the second housing 52 and the circuit board 6, and hermetically connect the second housing 52 and the circuit board 6. At least part of the second chamber 521 forms the second cavity 502, which is an airtight cavity. The second housing 52 and the circuit board 6 are both located on the periphery of the second cavity 502.

In this application, since the first cavity 501 formed by the connection of the first housing 51 and the circuit board 6 is an open cavity, the first housing 51 only needs to be fixedly connected to the circuit board 6. This connection can be either hermetic or non-hermetic. However, the second housing 52 needs to connect with the circuit board 6 to form a closed second cavity 502. Therefore, the connection between the second housing 52 and the circuit board 6 must not only achieve fixation between the two but also meet the sealing requirements of the second cavity 502. As a result, the second housing 52 is hermetically connected to the circuit board 6.

Although embodiments of this application have been shown and described, those skilled in the art can understand that various changes, modifications, substitutions, and variations can be made to these embodiments without departing from the principles and spirit of this application. The scope of this application is defined by the claims and their equivalents.

## Claims

1. A detection assembly, **characterized by** comprising a lead electrode; and
a conductive terminal, including a first terminal end and a second terminal end, wherein the first terminal end has a first tip, the second terminal end has a second tip, along the extension direction of the conductive terminal, the lead electrode and the first terminal end are on the same side of the second tip, wherein the lead electrode includes a connection portion and an extension portion, the connection portion is connected to the first terminal end, and the extension portion extends from the connection portion in a direction away from the conductive terminal,
wherein along the extension direction of the conductive terminal, the connection portion and the second terminal end are on the same side of the first tip; or, the connection portion and the second terminal end are on different sides of the first tip, and the connection portion is perpendicular to the extension direction of the conductive terminal,
wherein along the extension direction of the conductive terminal, the extension portion and the second terminal end are on the same side of the first tip; or, the extension portion and the second terminal end are on different sides of the first tip, and the extension portion is perpendicular to the extension direction of the conductive terminal.

2. The detection assembly according to claim 1, wherein, along the extension direction of the conductive terminal, the connection portion and the extension portion are on the same side of the first tip.

3. The detection assembly according to claim 1, wherein the connection portion and the extension portion are in the same plane, and the plane is parallel or perpendicular to the extension direction of the conductive terminal.

4. The detection assembly according to claim 1, wherein the connection part has a first connection end and a second connection end, the first connection end and the second connection end are located at both ends of the connection part along the height direction or width direction of the detection assembly, and the first connection end is closer to the first tip than the second connection end; and
the connection part has a connection point connected to the extension part, and the connection point is located at the first connection end; or, the connection point is located at the second connection end.

5. The detection assembly according to claim 1, wherein the connection portion includes a connection area for connecting with the conductive terminal, and the connection area is in the form of a line or a surface.

6. The detection assembly according to claim 6, wherein the conductive terminal includes a first conductive terminal and a second conductive terminal, and the lead electrode includes a first lead electrode and a second lead electrode;
the first lead electrode includes a first connection portion and a first extension portion, the first connection portion is connected to the first terminal end of the first conductive terminal, and the first extension portion extends from the first connection portion in a direction away from the first conductive terminal;
the second lead electrode includes a second connection portion and a second extension portion, the second connection portion is connected to the first terminal end of the second conductive terminal, and the second extension portion extends from the second connection portion in a direction away from the second conductive terminal; and
the first extension portion is located on the side of the first conductive terminal facing the second conductive terminal, and the second extension portion is located on the side of the second conductive terminal facing the first conductive terminal.

7. The detection assembly according to claim 6, wherein the detection assembly includes a detection element, and the detection element is electrically connected to the lead electrode;
the first extension portion includes a first connection segment and a second connection segment that are connected to each other, the first connection segment is connected to the detection element, the second connection segment extends from the first connection segment in a direction away from the detection element, and the second connection segment is connected to the first connection portion;
the second extension portion includes a third connection segment and a fourth connection segment that are connected to each other, the third connection segment is connected to the detection element, the fourth connection segment extends from the third connection segment in a direction away from the detection element, and the fourth connection segment is connected to the second connection portion; and
the first connection segment is parallel to the third connection segment.

8. The detection assembly according to claim 6, wherein the detection assembly includes a detection element, the first lead electrode and the second lead electrode are in the same plane, and at least part of the surface of the detection element is parallel to the plane.

9. The detection assembly according to claim 6, wherein the first lead electrode is axisymmetric to the second lead electrode, or the first lead electrode is centrosymmetric to the second lead electrode.

10. The detection assembly according to claim 1, wherein the detection assembly includes a detection element, and the detection element is electrically connected to the lead electrode;
the detection element and the first terminal end are on the same side of the second tip, the detection element has a first side and a second side, and the first side and the second side are on different sides of the detection element along the extension direction of the conductive terminal; and
along the extension direction of the conductive terminal, both the first side and the second side are on the same side of the first tip as the second terminal end; or, the first side and the second terminal end are on different sides of the first tip, the second side and the second terminal end are on different sides of the first tip, and the second side is flush with the first tip; or, the first side and the second side are on different sides of the first tip.

11. A sensor, comprising a detection assembly including a detection element (1), a lead electrode (2) connected to the detection element (1), and a conductive terminal (3) connected to the lead electrode (2),
wherein,
the conductive terminal includes a first terminal end and a second terminal end, the first terminal end has a first tip, the second terminal end has a second tip, along the extension direction of the conductive terminal, the lead electrode and the first terminal end are on the same side of the second tip , the lead electrode includes a connection portion and an extension portion, the connection portion is connected to the first terminal end, and the extension portion extends from the connection portion in a direction away from the conductive terminal;
along the extension direction of the conductive terminal, the connection portion and the second terminal end are on the same side of the first tip; or, the connection portion and the second terminal end are on different sides of the first tip, and the connection portion is perpendicular to the extension direction of the conductive terminal;
along the extension direction of the conductive terminal, the extension portion and the second terminal end are on the same side of the first tip; or, the extension portion and the second terminal end are on different sides of the first tip, and the extension portion is perpendicular to the extension direction of the conductive terminal;
a housing, including a chamber, wherein the detection element, the lead electrode, and the first terminal end are all located within the chamber, and the second terminal end is located outside the chamber; and
a circuit board connected to the housing and electrically connected to the detection assembly.

12. The sensor according to claim 11, wherein the detection element and the first terminal end are on the same side of the second tip, the detection element has a first side and a second side, and along the extension direction of the conductive terminal, the first side and the second side are on different sides of the detection element along the extension direction of the conductive terminal; and
along the extension direction of the conductive terminal, both the first side and the second side are on the same side of the first tip as the second terminal end; or, the first side and the second terminal end are on different sides of the first tip, the second side and the second terminal end are on different sides of the first tip, and the second side is flush with the first tip ; or, the first side and the second side are on different sides of the first tip.

13. The sensor according to claim 11, wherein the detection assembly includes a base, the conductive terminal passes through the base, the conductive terminal is fixedly connected to the base, the conductive terminal is fixedly connected to the circuit board, the detection element is electrically connected to the lead electrode, the lead electrode is electrically connected to the conductive terminal, and the conductive terminal is electrically connected to the circuit board; and
the housing is directly connected to the base, and the housing is fixedly connected or limitedly connected to the base; or, the housing is directly connected to the circuit board, and the housing is fixedly connected or limitedly connected to the circuit board, the base is located inside the housing, and there is a gap between at least part of the housing and the base.

14. The sensor according to claim 13, wherein the housing has a peripheral wall, the peripheral wall is located around the chamber, the housing has a third side and a fourth side, along the thickness direction of the circuit board, the third side is farther from the circuit board than the fourth side; and
the fourth side has a flanged part, the flanged part extends from the peripheral wall in a direction away from the housing, at least part of the flanged part is welded or bonded to the base or the circuit board.

15. The sensor according to claim 13, wherein the base includes a first support platform and a second support platform, the first support platform is connected to the second support platform, and the conductive terminal passes through the first support platform and the second support platform;
along the extension direction of the conductive terminal, the projection of the first support platform on the second support platform is within the outer contour of the second support platform, and both the first support platform and the second support platform are welded or bonded to the housing.

16. The sensor according to claim 11, wherein the sensor has a first cavity and a second cavity, the first cavity is in gas communication with the external environment of the sensor, and the second cavity is an airtight cavity;
the detection assembly includes a first detection assembly and a second detection assembly, the first detection assembly includes a first detection element, the second detection assembly includes a second detection element, the first detection element is located in the first cavity, and the second detection element is located in the second cavity; and
the housing includes a first housing and a second housing, the first housing is located around the first cavity, the second housing is located around the second cavity, the first housing and the second housing are separate from each other, and there is a gap between the first housing and the second housing.

17. The sensor according to claim 16, wherein the first housing has a first chamber, at least part of the first chamber forms the first cavity, the first cavity has a first opening, the first opening is in gas communication with the first cavity and the external environment of the sensor, the second housing has a second chamber, and at least part of the second chamber forms the second cavity;
the detection assembly includes a base, the conductive terminal passes through the base, the conductive terminal is fixedly connected to the base, the conductive terminal is fixedly connected to the circuit board, and the conductive terminal is electrically connected to the circuit board; and
the base includes a first base and a second base, both the first housing and the first base are located around the first cavity; both the second housing and the second base are located around the second cavity, the second housing is hermetically connected to the second base; or, the circuit board is located around the first cavity and the second cavity, the first base is located in the first cavity, the second base is located in the second cavity, and the second housing is hermetically connected to the circuit board.

18. The sensor according to claim 17, wherein the first housing has a second opening, the circuit board covers the second opening, along the height direction of the first housing, the first opening and the second opening are located on different sides of the first housing; and
the second housing has a third opening, the second housing has a wall surface located around the third opening, the circuit board covers the third opening, and along the height direction of the sensor, the projection of the wall surface on the circuit board is within the outer contour of the circuit board.

19. The sensor according to claim 17, wherein the base has a first central axis, the housing has a second central axis, and the first central axis coincides with the second central axis;
the first central axis of the first base passes through the first opening.

20. A manufacturing method for a sensor, comprising the following steps:
providing a detection assembly, a housing, and a circuit board, wherein the detection assembly includes a detection element, a conductive part, and a base, the detection element is connected to the conductive part, and the conductive part is connected to the base;
connecting the detection assembly to the circuit board;
fitting the housing over at least part of the detection assembly, connecting the housing to the circuit board.
